# EUROPEAN PATENT APPLICATION

(11) **EP 4 201 362 A1**
(43) Date of publication of application: **28.06.2023**
(21) Application number: 22157683.8
(22) Date of filing: 21.02.2022
(51) Int. Cl.: A61B 34/20, A61B 34/30, A61B 90/00, A61B 34/32

(54) **FLUOROSCOPIC IMAGE GUIDED ROBOTIC BLOOD VESSEL CANNULATION**

(30) Priority: 22.12.2021 US 202163292934 P
(71) Applicant: Koninklijke Philips N.V., 5656 AG Eindhoven (NL)
(72) Inventor: OLIVAN BESCOS, Javier, Eindhoven (NL); BALICKI, Marcin A, Eindhoven (NL)
(74) Representative: Philips Intellectual Property & Standards

(57) **Abstract**

Various embodiments of a vessel cannulation system employs an interventional robot (110) and a vessel cannulation controller (80) for a blood vessel cannulation of a target blood vessel by an endovascular instrument (10) navigational within a transitory blood vessel having the target blood vessel branching therefrom. In operation, the vessel cannulation controller (80) (i) defines, within an image space, a virtual wall of the transitory blood vessel having a virtual entryway into the target blood vessel, (ii) commands the interventional robot (110) to execute a positional wall sampling of the transitory blood vessel by the distal section of the endovascular instrument (10), and (iii) detects the blood vessel cannulation of the target blood vessel by the distal section of the endovascular instrument (10) through the virtual entryway of the virtual wall during the positional wall sampling of the transitory blood vessel by the distal section of the endovascular instrument (10).

## Description

### FIELD OF THE INVENTION

The present disclosure generally relates to a blood vessel cannulation during an endovascular intervention, particularly for treatment of a vascular disease (e.g., stroke, peripheral artery disease, abdominal aortic aneurysm, carotid artery disease, arteriovenous malformation, critical limb-threatening ischemia, pulmonary embolism, deep vein thrombosis, chronic venous insufficiency and varicose veins). The present disclosure specifically relates to a robotic blood vessel cannulation involving a fluoroscopic image guided feedback of a systematic positional wall sampling of a blood vessel by an endovascular instrument within the blood vessel.

### BACKGROUND OF THE INVENTION

Endovascular interventionalists use a guidewire co-axially aligned within a guide catheter to navigate a vasculature a human or an animal to a treatment site. A typical process to cannulate a blood vessel involves a set of discrete maneuvers coordinating the motion of the guidewire and the guide catheter to deposit the guidewire in a target vascular branch, over which the guide catheter is advanced to the treatment site. For experienced endovascular interventionalists, such skill for coordinating the motion of the guidewire and the guide catheter is learned through observation, and trial-and-error over many years. However, this is not the case for novice endovascular interventionalists, especially in cases that are considered difficult due to tortuous anatomy and/or difficult image feedback. It not only requires superb image interpretation abilities to navigate the 3D vascular anatomy, particularly when utilizing 2D perspective fluoroscopy, but also trained hand-eye coordination to precisely manipulate the coaxial aligned guidewire and guide catheter simultaneously.

Common endovascular wire-maneuver is cannulating a side branch(renal) from a large blood vessel (aorta). One of the steps in the process is to align the guide catheter so the distal section points are in the direction of the target blood vessel and then advance the guidewire into the branching blood vessel, followed by advancing the guide catheter over the guidewire, thus completing the cannulation process. As currently practiced, endovascular interventionalists randomly attempt to cannulate the branching blood vessel by traversing the wall of the large blood vessel with the guidewire/catheter assembly while actively "helicoptering" the guidewire with the hope of landing it inside the branching blood vessel. This trial-and-error approach is frustrating and can add up to 15+ minutes or more of wasted time or require an expert to take over.

### SUMMARY OF THE INVENTION

The present disclosure provides an improved endovascular intervention workflow by assisting in navigating an endovascular instrument within a transitory blood vessel into a target blood vessel branching from the transitory blood vessel by robotic means with high precision and speed. The present disclosure utilizes systematic positional wall sampling of the transitory blood vessel involving translating and/or rotating the endovascular instrument within the transitory blood vessel to one or more sampling positions and at each wall sample position, attempting to enter a target branch ostium by controlling a traversal of the guidewire across the transitory blood vessel, especially in cases where x-ray perspective does not capture the location of the target branch ostium. The present disclosure achieves this with fluoroscopic imaging synchronized with servo control of the endovascular instrument.

One embodiment of the present disclosure is a vessel cannulation system for a blood vessel cannulation of a target blood vessel by an endovascular instrument navigational within a transitory blood vessel.

The vessel cannulation system an interventional robot connectable to a proximal section of the endovascular instrument. When connected to the proximal section of the endovascular instrument, the interventional robot is configured to navigate the distal section of the endovascular instrument within the transitory blood vessel.

The vessel cannulation system further employs a vessel cannulation controller configured to (1) define, within an-ray image space (e.g., X-ray) encompassing the target blood vessel branching from the transitory blood vessel, a virtual wall of the transitory blood vessel having a virtual entryway into the target blood vessel, (2) command the interventional robot to execute a positional wall sampling of the transitory blood vessel by the distal section of the endovascular instrument, and (3) detect the blood vessel cannulation of the target blood vessel by the distal section of the endovascular instrument through the virtual entryway of the virtual wall during the positional wall sampling of the transitory blood vessel by the distal section of the endovascular instrument.

A second embodiment of the present disclosure is a vessel cannulation controller employing a non-transitory machine-readable storage medium encoded with instructions for execution by one or more processors for controlling a blood vessel cannulation of a target blood vessel by an endovascular instrument having a proximal section connected to an interventional robot and a distal section navigational within a transitory blood vessel having the target blood vessel branching therefrom.

The non-transitory machine-readable storage medium includes the instructions to (1) define, within an image space (e.g., X-ray) encompassing the target blood vessel branching from the transitory blood vessel, a virtual wall of the transitory blood vessel having a virtual entryway into the target blood vessel, (2) command the interventional robot to execute a positional wall sampling of the transitory blood vessel by the distal section of the endovascular instrument, and (3) detect the blood vessel cannulation of the target blood vessel by the distal section of the endovascular instrument through the virtual entryway of the virtual wall during the positional wall sampling of the transitory blood vessel by the distal section of the endovascular instrument.

A third embodiment of the present disclosure is a vessel cannulation method executable by a vessel cannulation controller for a blood vessel cannulation of a target blood vessel by an endovascular instrument having a proximal section connected to an interventional robot and a distal section navigational within a transitory blood vessel having the target blood vessel branching therefrom.

The cannulation method involves the vessel cannulation controller (1) defining, within an image space (e.g., X-ray) encompassing the target blood vessel branching from the transitory blood vessel, a virtual wall of the transitory blood vessel having a virtual entryway into the target blood vessel, (2) commanding the interventional robot to execute a positional wall sampling of the transitory blood vessel by the distal section of the endovascular instrument, and (3) detecting the blood vessel cannulation of the target blood vessel by the distal section of the endovascular instrument through the virtual entryway of the virtual wall during the positional wall sampling of the transitory blood vessel by the distal section of the endovascular instrument.

For all embodiments of the present disclosure, the endovascular instrument may include an inner endovascular device (e.g., a guidewire) coaligned within an outer endovascular device (e.g., a guide catheter). When connected to the proximal sections of both endovascular devices, the interventional robot is configured to individually or collectively navigate the distal sections of the endovascular devices as commanded by the vessel cannulation controller.

Additionally, for all X-ray embodiments of the present disclosure, a detection of the blood vessel cannulation of the target blood vessel through the virtual barrier by the distal section of the endovascular instrument may be confirmed or tested by the vessel cannulation controller from (a) an X-ray imaging of a contrast injection into the bloods vessels and/or (2) an X-ray imaging of a lateral perspective of the target blood vessel relative to the transitory blood vessel.

For purposes of the description and claims of the present disclosure:
(1) terms of the art of the present disclosure including, but not limited to, "endovascular intervention" and "interventional robot" are to be interpreted as known in the art of the present disclosure and as exemplary described in the present disclosure;
(2) the term "blood vessel cannulation" broadly encompasses any type of cannulation, as known in the art of the present disclosure and hereinafter conceived, of a blood vessel, a fenestrated stent or any type of tubular device within a vasculature;
(3) the term "endovascular instrument" broadly encompasses any instrument, as known in the art of the present disclosure and hereinafter conceived, including one or more endovascular devices that are navigational within a vasculature during an endovascular intervention, particularly for treatment of a vascular disease (e.g., stroke, peripheral artery disease, abdominal aortic aneurysm, carotid artery disease, arteriovenous malformation, critical limb-threatening ischemia, pulmonary embolism, deep vein thrombosis, chronic venous insufficiency and varicose veins);
(4) the term "guidewire" broadly encompasses wires and springs, as known in the art of the present disclosure and hereinafter conceived, that are navigational through a vasculature during an endovascular intervention;
(5) the term "guide catheter" broadly encompasses flexible tubes, as known in the art of the present disclosure and hereinafter conceived, that are navigational through a vasculature during an endovascular intervention;
(6) the term "transitory blood vessel" broadly encompasses any blood vessel of a vasculature, excluding a treatment site of a vasculature, that must be navigated to reach the treatment site;
(7) the term "target blood vessel" broadly encompasses any blood vessel of a vasculature branching from a transitory blood vessel and including or excluding a treatment site;
(8) the term "wall sample position" broadly encompasses a specific location and a specific orientation of a distal section of an endovascular instrument within a transitory blood vessel;
(9) the term "positional wall sampling" broadly encompasses a positioning of a distal section of an endovascular instrument at one or more wall sample positions within a transitory blood vessel for purposes of traversing the distal section of the endovascular instrument across the transitory blood vessel in a direction of a physical wall of the transitory wall in an effort to enter an ostium of a target blood vessel, such as, for example, traversing a distal section of a guidewire extending out of a guide catheter across the transitory blood vessel in a direction of a physical wall of the transitory wall;
(10) the term "virtual wall" of a transitory blood vessel broadly encompasses any type of computed simulation, as known in the art of the present disclosure or hereinafter conceived, of the physical wall of the transitory blood vessel including an entryway into the target blood vessel;
(11) the term "virtual entryway" into a target blood vessel broadly encompasses any type of computed simulation, as known in the art of the present disclosure or hereinafter conceived, of an ostium into the target blood vessel;
(12) the phrase "define a virtual wall" and "defining a virtual wall" broadly encompasses a formulation of one or more metrics for delineating the virtual wall within X-ray image space (e.g., data fitting, force, distance and/or shape) that may be supplemented by user manipulation and/or image processing of an X-ray image of the blood vessels as exemplary shown herein;
(13) the term "image space" broadly encompasses the two-dimensional, three-dimensional or four dimensional space encompassing an image of the blood vessels (e.g., X-ray, ultrasound, MRI, CT, endoscopic, etc.);
(14) the term "controller" broadly encompasses all structural configurations, as understood in the art of the present disclosure and as exemplary described in the present disclosure, of main circuit board or integrated circuit for controlling an application of various aspects of the present disclosure as exemplary described in the present disclosure. The structural configuration of the controller may include, but is not limited to, processor(s), computer-usable/computer readable storage medium(s), an operating system, application module(s), peripheral device controller(s), slot(s) and port(s). A controller may be housed within or linked to a workstation. Examples of a "workstation" include, but are not limited to, an assembly of one or more computing devices, a display/monitor, and one or more input devices (e.g., a keyboard, joysticks and mouse) in the form of a standalone computing system, a client computer of a server system, a desktop, a laptop or a tablet;
(15) the term "application module" broadly encompasses an application incorporated within or accessible by a controller consisting of an electronic circuit (e.g., electronic components and/or hardware) and/or an executable program (e.g., executable software stored on non-transitory computer readable medium(s) and/or firmware) for executing a specific application; and
(16) the terms "data" and "signal" broadly encompasses all forms of a detectable physical quantity or impulse (e.g., voltage, current, or magnetic field strength) as understood in the art of the present disclosure and as exemplary described in the present disclosure for transmitting information and/or instructions in support of applying various aspects of the present disclosure as subsequently described in the present disclosure. Data/signal communication components of the present disclosure may involve any communication method as known in the art of the present disclosure including, but not limited to, data/signal transmission/reception over any type of wired or wireless datalink/signal link and a reading of data/signal uploaded to a computer-usable/computer readable storage medium.

The foregoing embodiments and other embodiments of the inventions of the present disclosure as well as various structures and advantages of the inventions of the present disclosure will become further apparent from the following detailed description of various embodiments of the inventions of the present disclosure read in conjunction with the accompanying drawings. The detailed description and drawings are merely illustrative of the inventions of the present disclosure rather than limiting, the scope of the inventions of the present disclosure being defined by the appended claims and equivalents thereof.

### BRIEF DESCRIPTION OF THE DRAWINGS

FIG. 1 illustrates an exemplary embodiment of a blood vessel cannulation system in accordance with various aspects of the present disclosure.
FIG. 2A illustrates an exemplary endovascular instrument within a transitory blood vessel relative to an "out of plane" target blood vessel as known in the art of the present disclosure.
FIG. 2B illustrates an exemplary ostium of the "out of plane" target blood vessel as illustrated in FIG. 2A.
FIG. 3 illustrates an exemplary planar X-ray image showing a range of a translating motion and a traversal motion of an endovascular instrument within a transitory blood vessel relative to a target blood vessel in accordance with various aspects of the present disclosure.
FIG. 4 illustrates an exemplary embodiment of a vessel cannulation control network in accordance with the present disclosure.
FIG. 5 illustrates a flowchart representative of vessel cannulation method in accordance with various aspects of the present disclosure.
FIG. 6 illustrates an exemplary positional wall sampling in accordance with the present disclosure.
FIG. 7A illustrates an exemplary embodiment of a best fit metric applied to the positional wall sampling of FIG. 6 in accordance with the present disclosure.
FIG. 7B illustrates an exemplary embodiment of a force metric applied to the positional wall sampling of FIG. 6 in accordance with the present disclosure.
FIG. 7C illustrates an exemplary embodiment of a distance metric applied to the positional wall sampling of FIG. 6 in accordance with the present disclosure.
FIG. 7D illustrates an exemplary embodiment of a shape metric applied to the positional wall sampling of FIG. 6 in accordance with the present disclosure.
FIG. 8 illustrates an exemplary embodiment of the vessel cannulation system illustrated in FIG. 1 in accordance with various aspects of the present disclosure.
FIG. 9A illustrates an exemplary embodiment of a flowchart representative of a vessel cannulation method illustrated in FIG. 4 in accordance with various aspects of the present disclosure.
FIG. 9B illustrates an exemplary embodiment of a flowchart representative of a positional wall sampling method in accordance with various aspects of the present disclosure.
FIGS. 10A-10H illustrate a first exemplary execution of the positional wall sampling method illustrated in FIG. 9B.
FIGS. 11A-11D illustrate a second exemplary execution of the positional wall sampling method illustrated in FIG. 9B.
FIG. 12 illustrates an exemplary embodiment of a vessel cannulation controller in accordance with various aspects of the present disclosure.

### DETAILED DESCRIPTION OF THE PREFERRED EMBODIMENTS

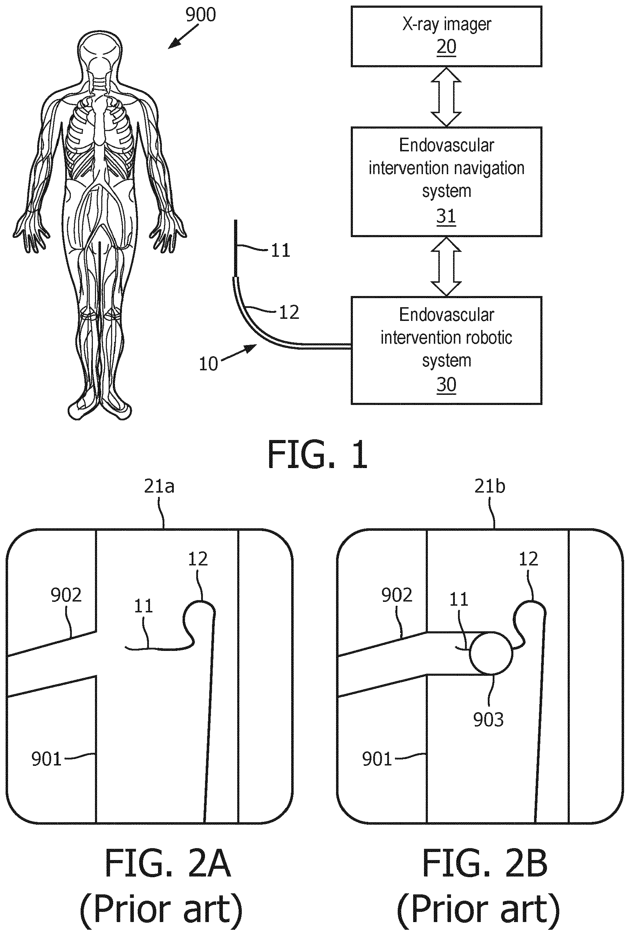

To facilitate an understanding of various inventive aspects of the present disclosure, the following description of FIGS. 1-3 teaches embodiments of a blood vessel cannulation of the present disclosure. From this description, those having ordinary skill in the art will appreciate how to apply the various aspects of the present disclosure for making and using additional embodiments of blood vessel cannulation of the present disclosure.

Referring to FIG. 1, a blood vessel cannulation of the present disclosure is performed during an endovascular intervention of a patient 900, particularly for treatment of a vascular disease by an endovascular instrument 10 including one or more endovascular devices that are navigational within a vasculature during an endovascular intervention (e.g., stroke, peripheral artery disease, abdominal aortic aneurysm, carotid artery disease, arteriovenous malformation, critical limb-threatening ischemia, pulmonary embolism, deep vein thrombosis, chronic venous insufficiency and varicose veins).

In the illustrated embodiment throughout the figures, endovascular instrument 10 is shown as including a co-alignment of a guidewire 11 within a guide catheter 12 as known in the art of the present disclosure, and the present disclosure is described in the context of the co-alignment of guidewire 11 within guide catheter 12. Nonetheless, those having ordinary skill will appreciate alternative embodiments of endovascular instrument 10 in accordance with the present disclosure.

Still referring to FIG. 1, patient 900 has a vast vasculature network of blood vessels whereby endovascular instrument 10 is navigated within the vasculature network of patient 900 to a treatment site via an endovascular intervention robotic system 32 as commanded by an endovascular intervention navigation system 31 receiving fluoroscopic feedback from a X-ray imager 20.

In practice, X-ray imager 20 is any system for generating X-ray/fluoroscopic images of a vasculature as known in the art of the present disclosure or hereinafter conceived, such as, for example, (1) Philips Azurion fixed C-Arm X-ray, (2) Philips Zenition Mobile X-ray and (3) CombiDiagnost R90 fluoroscopy system. Furthermore, any other type of medical imager for generating images of a vasculature may be utilized, as known in the art of the present disclosure or hereinafter conceived, for generating images of a vasculature may be utilized alternative to X-ray imager 20 (e.g., an ultrasound imager, a MRI imager, a CT imager and an endoscope).

In practice, endovascular intervention robotic system 30 is any robotic system for navigating a endovascular instrument within vasculature network of blood vessels as known in the art of the present disclosure or hereinafter conceived, such as, for example, (1) Corindus CorPath GRX , (2) RoboCath R1 and (3) Stereotaxis Genesis RMN robot systems.

For an endovascular instrument embodiment being a single endovascular device, robotic system 30 will be connected to a proximal section of the single endovascular device for navigating a distal section of the single endovascular device as known in the art of the present disclosure. For example, endovascular instrument 10 may be guidewire 11 having a proximal section connected to robotic system 30 for navigating the distal section of guidewire 11 as known in the art of the present disclosure.

For an endovascular instrument embodiment including two or more endovascular devices, robotic system 30 will be connected to the proximal sections of the endovascular devices for individually or collectively navigating the distal sections of the endovascular devices as known in the art of the present disclosure. For example, as illustrated in FIG. 1, guidewire 11 and guide catheter 12 have proximal sections connected to robotic system 30 for individually or collectively navigating the distal sections of guidewire 11 and guide catheter 12 as known in the art of the present disclosure.

Still referring to FIG. 1, in practice, endovascular intervention navigation system 31 incorporates an improved endovascular intervention workflow assisting in a navigation of endovascular instrument 10 from a transitory blood vessel into a target blood vessel branching from the transitory blood vessel via endovascular intervention robotic system 30 with high precision and speed.

Generally, endovascular intervention navigation system 31 utilizes systematic positional wall sampling of the transitory blood vessel involving translating and/or rotating the endovascular instrument within the transitory blood vessel to one or more sampling positions and at each wall sample position, attempting to enter a target branch ostium by controlling a traversal of an endovascular device across the transitory blood vessel, especially in cases where an image perspective does not capture the location of the target branch ostium. This achieved with fluoroscopic imaging from X-ray imager 20 that is synchronized with servo control of endovascular intervention robotic system 30 by endovascular intervention navigation system 31.

More particularly, locating a target vessel's ostium (entry orifice) for cannulation is typically difficult because the target branch may be located in a different plane than the endovascular instrument 10, which may not be apparent in 2D fluoro imaging by X-ray imager 20, with or without a technique for visualizing the blood vessels (e.g., Digital Subtraction Angiography (DSA)). Even with a technique like DSA, maneuvering the guidewire 11/guide catheter 12 combination to deposit guidewire 11 into a target vessel is challenging due to its proportionally small diameter of the target vessel's ostium and random spatial variation (interpersonal) of the vessel entry location, and poor controllability of guidewire 11 and guide catheter 12.

A more extreme problem is the cannulation of target vessel's ostium that is significantly "out of plane". Due to a projective nature of 2D X-ray images, when a target vessel branches off in a direction perpendicular to the image plane, it is difficult or impossible to locate the target vessel's ostium in the 2D X-ray image. For example, FIG. 2A illustrates an X-ray image 21a of an "out of plane" target vessel where a distal section of a guide catheter 12 appears to align with an ostium of target vessel 902. However, as shown in FIG. 2B, a tip of guide catheter 12 is actually contacting the wall of the transitory vessel 901, because a true ostium 903 of the target vessel 902 is actually in the center of the transitory vessel 901 as shown in X-ray image 21b. Thus the distal section of the guide catheter 12 should be oriented in or out of plane in the center to have a chance of cannulating the target vessel 902.

To address the cannulation of ostium 903 of the target vessel 902 is "out of plane" of an X-ray image, particularly when ostium 903 of the target vessel 902 is significantly "out of plane", the present disclosure provides a positional wall sampling of a transitory vessel by robotically driven coaxial devices where one of the devices is translated while the other is oscillated back and forth, where the extents of the oscillating device in x-ray images define a virtual boundary, and the motion stops when the boundary is breached by the oscillating device.

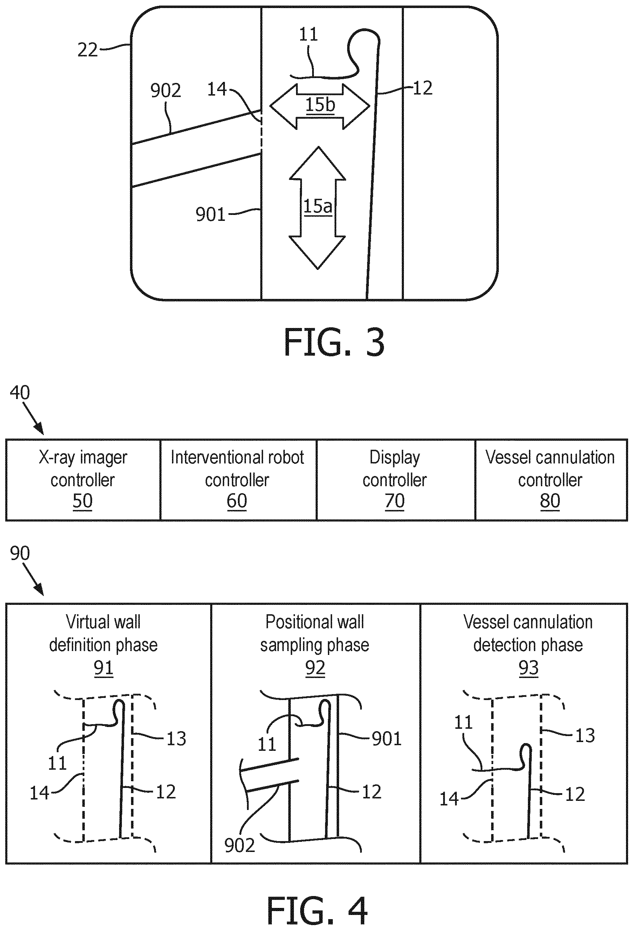

More particularly, as shown in FIG. 3, an X-ray image 22 is taken of a target blood vessel 902 branching from transitory blood vessel 901. Within the X-ray image space, a virtual wall of transitory blood vessel 901 having a virtual entryway 14 into the target blood vessel 902 is defined as will be further exemplarily described in the present disclosure.

Subsequently, as will be further exemplarily described in the present disclosure, a positional wall sampling of the transitory blood vessel 901 involves a linear translation motion 15a and/or a rotation (not shown) of by the distal sections of guidewire 11 and guide catheter 12 collectively within transitory vessel 901 between sampling positions, and at each sampling position, a traversal motion 15b of the guidewire 11 relative to guide catheter 12 across transitory vessel 901 whereby guidewire 11 will either contact the physical wall of transitory blood vessel 901 or enter target blood vessel 902.

As will be further exemplarily described in the present disclosure, a blood vessel cannulation of target blood vessel 902 by guidewire 11 through the virtual entryway 14 of the virtual wall is detected during the positional wall sampling of the transitory blood vessel 902.

To further facilitate an understanding of various inventive aspects of the present disclosure, the following description of FIGS. 4-7D teaches embodiments of a control network for controlling vessel cannulation methods of the present disclosure. From this description, those having ordinary skill in the art will appreciate how to apply the various aspects of the present disclosure for making and using additional embodiments of control networks for controlling vessel cannulation methods of the present disclosure.

Referring to FIG. 4, a control network 40 includes an X-ray imager controller 50, an intervention robot controller 60, a display controller 70 and a vessel cannulation controller 80.

In practice, X-ray imager controller 50 controls X-ray image acquisitions by X-ray imager 20 (FIG. 1) as known in the art of the present disclosure or hereinafter conceived.

In various embodiments, X-ray imager controller 50 may be configured to improve the visualization of guidewire 11 and guide catheter 12 within an X-ray image using a known technique, such as, for example, Digital Subtraction Angiography (DSA). For these embodiments, the positional wall sampling may be used to update the DSA based on estimate vessel wall location, and a heatmap may be generated that shows possible guidewire motion boundary in the X-ray images.

Furthermore, for each X-ray image, X-ray image controller 50 may be configured to parametrize (or segment) features of guidewire 11 and/or guide catheter 12 to identify shape or curvature (e.g. line segment representation, curvature, spine, or any parametric representation of the shape) by using various techniques known in the art of the present disclosure or hereinafter conceived, such as, for example, Artificial Intelligence segmentation (e.g. U-Net deep learning segmentation technique) or any known type of segmentation method including thresholding, hessian-based vesselness filter, active contours, etc.

Still referring to FIG. 4, in practice, intervention robot controller 60 controls a navigation of an endovascular instrument by an intervention robot within a vasculature network as known in the art of the present disclosure or hereinafter conceived.

For guide wire 11 and guide catheter 12), such navigation controls include a translation and a rotation of guide wire 11 and guide catheter 12 collectively within a blood vessel as known in the art of the present disclosure, and an extension and retraction of guidewire 11 relative to guide catheter 12 as known in the art of the present disclosure. Such navigation controls may further include a flexion of the distal sections of guide wire 11 and guide catheter 12 collectively.

Furthermore, a joint space of the intervention robot may be utilized to establish a regularized grid pattern in the robot control parameter space (e.g., joint angles) for a basic implementation of a positional wall sampling, or a probabilistic method that distributes the cannulation attempt configurations (e.g., joint angles) in a multidimensional Gaussian may be utilized for the positional wall sampling.

Still referring to FIG. 4, in practice, display controller 70 controls a display of X-ray images and associated graphical user interfaces (GUIs) as known in the art of the present disclosure or hereinafter conceived, such as, for example, GUIs that facilitate a user selection of a target vessel 902 illustrated within an X-ray image and optionally a delineation of virtual entryway 14 within the X-ray image as displayed.

Still referring to FIG. 4, in practice, vessel cannulation controller 80 controls an implementation of a vessel cannulation control 90 involving a virtual wall definition phase 91, a positional wall sampling phase 92, and vessel cannulation detection phase 93.

Virtual wall definition phase 91 encompasses vessel cannulation controller 80 defining, within an X-ray image space, a virtual wall 13 of transitory blood vessel 901 having a virtual entryway 14 into the target blood vessel 902.

Positional wall sampling phase 92 encompasses vessel cannulation controller 80 commanding interventional robot controller 60 to control an interventional robot connected to proximal sections of guidewire 11 and guide catheter 12 to translate and/or rotate the distal sections of guidewire 11 and guide catheter 12 collectively within transitory vessel 901 between sampling positions, and at each sampling position, to traverse guidewire 11 relative to guide catheter 12 across transitory vessel 901 whereby guidewire 11 will either contact the physical wall of transitory blood vessel 901 or enter target blood vessel 902.

Vessel cannulation detection phase 93 encompasses vessel cannulation controller 80 detecting when guidewire 11 entered target blood vessel 902 during positional wall sampling phase 92 applying a metric to the translational motion of guidewire 11 across transitory vessel 901 that is indicative of guidewire 11, at each sampling position, either contacting the physical wall of transitory blood vessel 901 or traversing through virtual entryway 14 into target blood vessel 902.

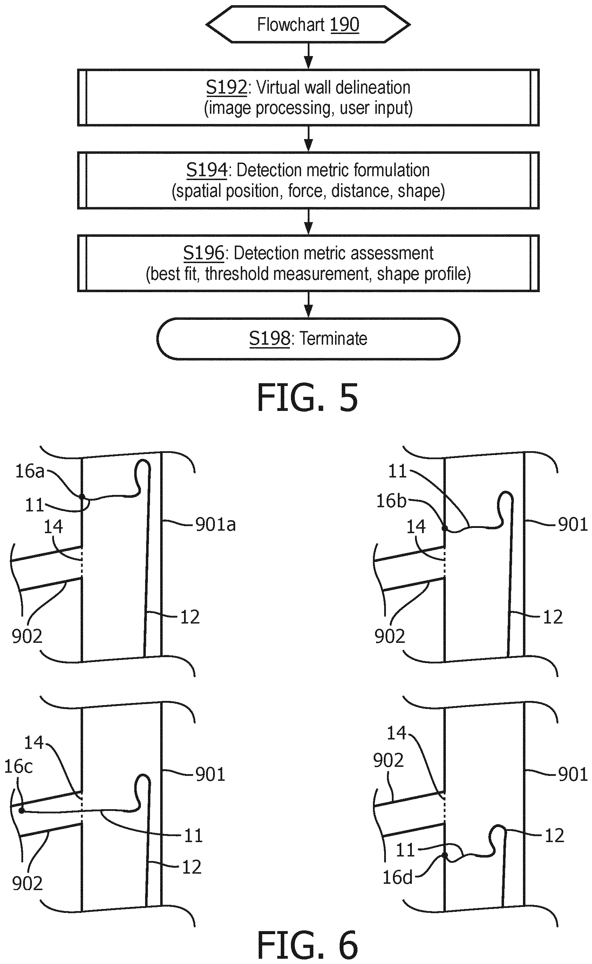

FIG. 5 illustrates a flowchart 190 represented an exemplarily embodiment of phases 91-93 as executed by vessel cannulation controller 80.

Referring to FIG. 5, upon initiation of the endovascular intervention, a stage S192 of flowchart 190 encompasses vessel cannulation controller 80 delineating the virtual wall including virtual entryway 14 within an initial X-ray image taken of target blood vessel 902 branching from transitory blood vessel 901 and of the distal sections of guidewire 11 and guide catheter 12 being within transitory blood vessel 901.

In one exemplary embodiment of stage S192, vessel cannulation controller 80 segments the vessels 901 and 902 in a DSA X-ray image, and detects the target vessel entry in the X-ray image space. Subsequently, vessel cannulation controller 80 constructs virtual entryway 14 within the X-ray image space using image processing techniques as known in the art of the present disclosure or hereinafter conceived (e.g., skeletonization, and T- intersection etc.) and extends the virtual wall from the virtual entryway 14.

In a second embodiment of stage S192, vessel cannulation controller 80 provides a user interface for enabling a user to delineate the location of the target vessel entry using one or more line segments in the X-ray image. For example, one or more line segment(s) and/or arc(s) may be placed the target vessel entry to represent virtual entryway 14 and another line segment may be placed across the proximal section of the target vessel to represent the ostium of the target vessel relative to the virtual entryway 14.

In a third embodiment of stage S192, vessel cannulation controller 80 may delineate virtual entryway 14 as a threshold during stage S194 as will be subsequently described in the present disclosure.

Still referring to FIG. 5, stage S194 of flowchart 140 encompasses vessel cannulation controller 80 formulating a metric that will be applied to the translational motion of guidewire 11 across transitory vessel 901 that is indicative of guidewire 11, at each sampling position, either contacting the physical wall of transitory blood vessel 901 or traversing through virtual entryway 14 into target blood vessel 902.

In one embodiment of stage S194, the metric is a spatial positioning measurement of a plurality of sampled points during the positional wall sampling of transitory blood vessel 902 that enables vessel cannulation controller 80 to determine one or more outliers representative of guide 11 entering target blood vessel 902 through virtual entryway 14 during the positional wall sampling of transitory blood vessel 901.

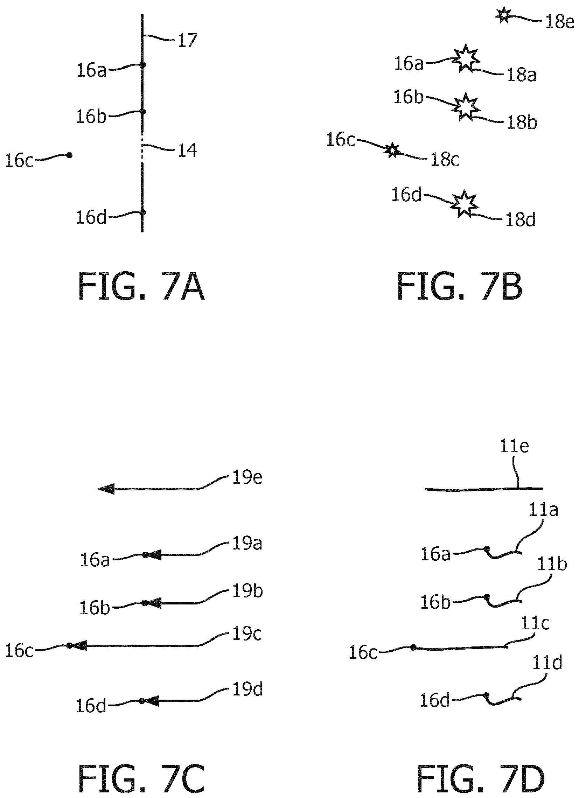

For example, FIG. 6 illustrates four (4) sample points 16a-16d during a positional wall sampling of transitory blood vessel 901. In stage S196 of flowchart 190, vessel cannulation controller 80 will delineate a line segment relative to virtual entryway 14 that will identify one or more outliers representative of a vessel canulation of target blood vessel 902, such as, for example, a line segment 17 shown in FIG. 7A that identifies sample point 16c as an outlier representative of guide 11 entering target blood vessel 902 through virtual entryway 14 during the positional wall sampling of transitory blood vessel 901.

Referring back to FIG. 5, in a second embodiment of stage S194, the metric is a force measurement of a plurality of sampled points during the positional wall sampling of transitory blood vessel 902 that enables vessel cannulation controller 80 to determine, at each sampling position, whether guidewire 11 contacted the physical wall of transitory blood vessel 901 or traversed through virtual entryway 14 into target blood vessel 902. In stage S196 of flowchart 190, vessel cannulation controller 80 will assess the measured force at each sample point to determine if guidewire 11 traversed through virtual entryway 14 into target blood vessel 902.

For example, using the four (4) sample points 16a-16d of FIG. 6, vessel cannulation controller 80 will establish a force threshold, particularly over a predefined traversal distance, whereby guidewire 11 is determined to have contacted the physical wall of transitory blood vessel 901 when the force measurement at a tip of guidewire 11 exceeds the force threshold and whereby guidewire 11 is determined to have traversed through virtual entryway 14 into target blood vessel 902 when the force measurement at a tip of guidewire 11 is below the force threshold. FIG. 7B exemplary illustrates force measurements 18a, 18b and 18d at respective sample points 16a, 16b and 16d exceeding a force threshold 18e, and a force measurement 18c at sample point 16c being below the force threshold 18e.

In practice, the force measurement may be directly measured, such as, for example, by a force sensor or a strain sensor in guidewire 11, or indirectly measured such as, by example, a force applied by actuators of the interventional robot to implement a predefined speed of the traversal of guidewire 11 across transitory blood vessel 901.

Referring back to FIG. 5, in a third embodiment of stage S194, the metric is a distance measurement of a plurality of sampled points during the positional wall sampling of transitory blood vessel 902 that enables vessel cannulation controller 80 to determine, at each sampling position, whether guidewire 11 contacted the physical wall of transitory blood vessel 901 or traversed through virtual entryway 14 into target blood vessel 902. In stage S196 of flowchart 190, vessel cannulation controller 80 will assess the measured distance at each sample point to determine if guidewire 11 traversed through virtual entryway 14 into target blood vessel 902.

For example, using the four (4) sample points 16a-16d of FIG. 6, vessel cannulation controller 80 will establish a distance threshold whereby guidewire 11 is determined to have contacted the physical wall of transitory blood vessel 901 when a vector magnitude is below the distance threshold, and whereby guidewire 11 is determined to have traversed through virtual entryway 14 into target blood vessel 902 when the a vector magnitude exceeds the distance threshold. FIG. 7C exemplary illustrates vectors 19a, 19b and 19d at respective sample points 16a, 16b and 16d being below a distance threshold 19e, and a vector 19c at sample point 16c exceeding the distance threshold 19e.

Referring back to FIG. 5, in a fourth embodiment of stage S194, the metric is a shape profile of guidewire 11 at a plurality of sampled points during the positional wall sampling of transitory blood vessel 902 that enables vessel cannulation controller 80 to determine, at each sampling position, whether guidewire 11 contacted the physical wall of transitory blood vessel 901 or traversed through virtual entryway 14 into target blood vessel 902. In stage S196 of flowchart 190, vessel cannulation controller 80 will assess the guidewire 11 at each sample point to determine if guidewire 11 traversed through virtual entryway 14 into target blood vessel 902.

For example, using the four (4) sample points 16a-16d of FIG. 6, vessel cannulation controller 80 will establish match a standard shape profile 11e whereby guidewire 11 is determined to have contacted the physical wall of transitory blood vessel 901 when guidewire 11 has a dissimilar shape profile compared to the standard shape profile, and whereby guidewire 11 is determined to have traversed through virtual entryway 14 into target blood vessel 902 when guidewire 11 has matching or similar shape profile. FIG. 7D exemplary illustrates shape profiles 11a, 11b and 11d at respective sample points 16a, 16b and 16d a dissimilar shape profile compared to the standard shape profile 11e, and a shape profile 11c at sample point 16c having a similar shape profile compared to standard shape profile 11e.

Referring back to FIG. 5, the examples of FIG. 7A-7D are simplified illustrations of a metric formulations/assessments. In practice, metric formulation/assessment can combine various factors, such as, for example, a force metric and a distance metric may be combined.

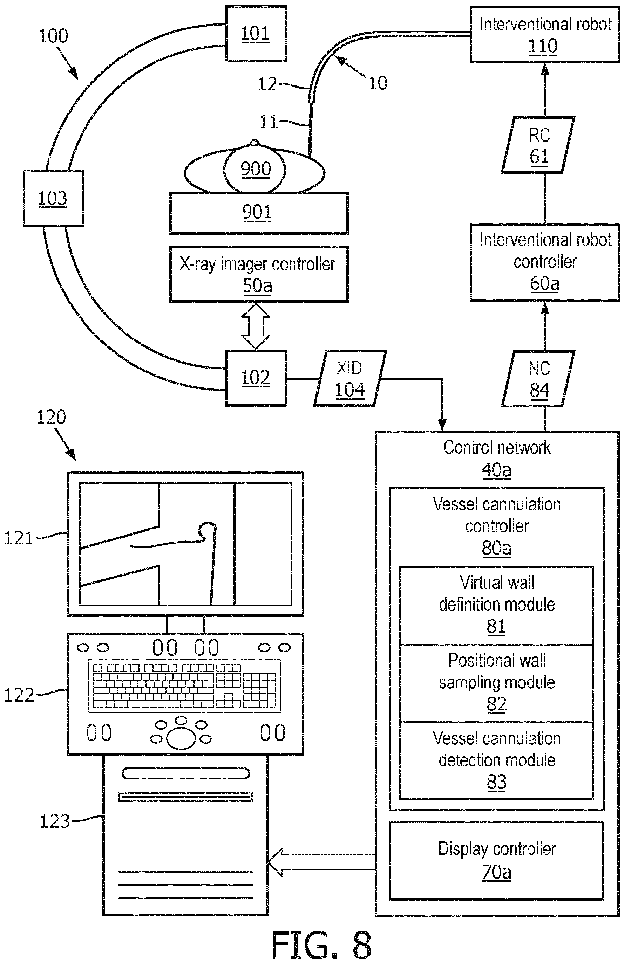

To facilitate a further understanding of various inventive aspects of the present disclosure, the following description of FIGS. 8-11 teaches embodiments of vessel cannulation system of the present disclosure. From this description, those having ordinary skill in the art will appreciate how to apply the various aspects of the present disclosure for making and using additional embodiments of vessel canulation systems of the present disclosure.

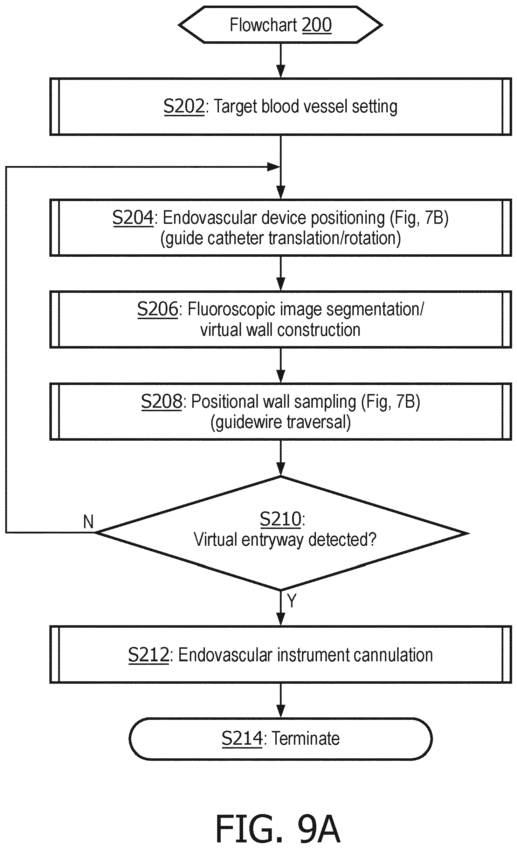

Referring to FIG. 8, a vessel cannulation system is shown for implementing a flowchart 200 as shown in FIG. 9A.

Referring to FIG. 8, an X-ray imager 100 includes a C-Arm 103 having an X-ray emitter 102 and an X-ray detector 101 for acquiring X-ray images 104. An intervention robot 110 is controlled by an intervention robot controller 60a via robot commands 61 to navigation a guidewire 11 and a guide catheter 12 within a vasculature of patient 900 on an operating table OT.

A workstation 120 includes a display 121, a keyboard 122 and a computer 123 incorporating a control network 40a including vessel cannulation controller 80a and display controller 70a.

Vessel cannulation controller 80a includes a virtual wall definition module 81 for defining the virtual wall 13 of the transitory blood vessel 901 having the virtual entryway 14 into the target blood vessel 902 as set forth in the description of FIGS. 4 and 5 of the present disclosure.

Vessel cannulation controller 80a further includes a positional wall sampling module 82, and a vessel cannulation detection module 83 for executing flowchart 200 of FIG. 9A as an exemplary embodiment of the positional wall sampling phase 92 and the vessel cannulation detection 93 of FIG. 4 of the present disclosure.

Referring to FIG. 9A, a target blood vessel setting stage S202 of flowchart 200 encompasses vessel cannulation controller 80a detecting operator input for vessel cannulation whereby controller 80a attempts to guide a positioning of X-ray imager 100 perpendicular to a target vessel to a highest degree detectable.

An endovascular instrument positioning stage S204 of flowchart 200 encompasses positional wall sampling module 82 commanding the intervention robot controller 60a to translate and/or rotate the guide catheter to a sample position relative to the target vessel.

A fluoroscopic image segmentation/virtual wall construction stage S206 of flowchart 200 encompasses positional wall sampling module 82 segmenting the guidewire and the guide catheter from an X-ray image and building a virtual wall boundary over the target vessel 902 as shown in the X-ray image.

A positional wall sampling stage S208 of flowchart 200 encompasses positional wall sampling module 82 commanding the intervention robot controller 60a to traverse the guide wire across the transitory vessel. If vessel cannulation detection module 83 ascertains the virtual wall has been breached during a stage S210 of flowchart 200, then a stage S212 of flowchart 200 encompasses positional wall sampling module 82commanding the intervention robot controller 60a to navigate the endovascular instrument into the located target vessel. Otherwise, if vessel cannulation detection module 83 ascertains the guidewire physically contacted the physical wall of the transitory vessel during a stage S210 of flowchart 200, then stages S204-S210 are repeated until the virtual wall has been breached by the guidewire.

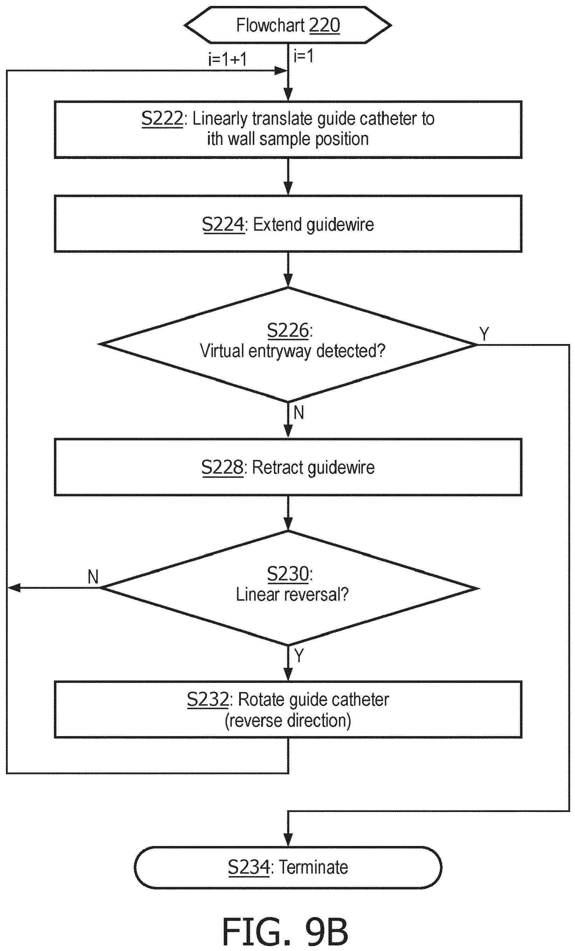

FIG. 9B illustrates a flowchart 220 representative of a stages S204, 208 and S210 loop of flowchart 200 of FIG. 9A.

Referring to FIG. 9B, a stage S222 of flowchart 200 encompasses a linearly translation of the guide catheter to a first wall sample position within the transitory vessel relative to the target vessel, and a stage S224 of flowchart 200 encompasses an extension of the guidewire out of the guide catheter across the transitory vessel.

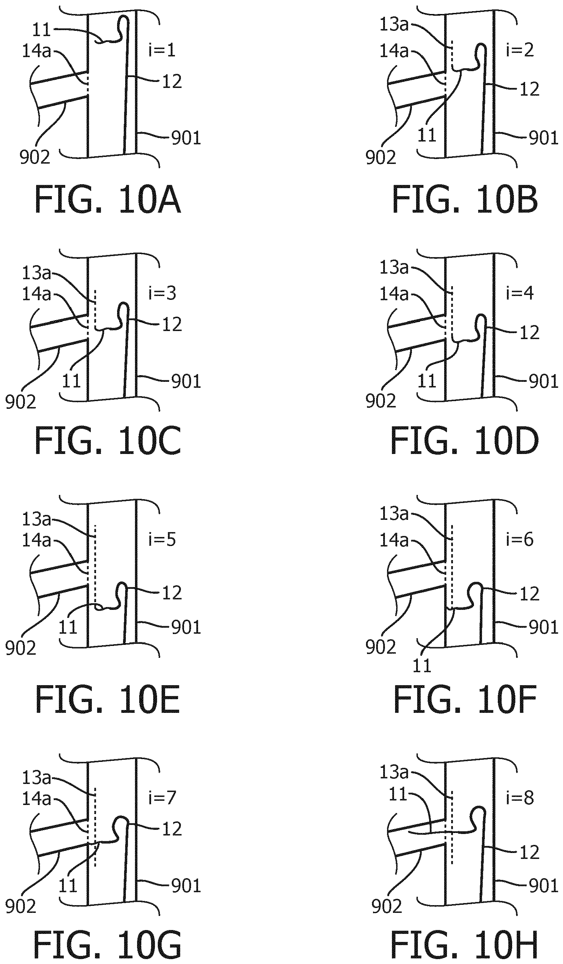

For example, FIG. 10A illustrates an exemplary translational positioning of guidewire 11 and guide catheter 12 to a first wall sample position within the transitory vessel 901 relative to the target vessel 902, and an extension of the guidewire 11 out of the guide catheter 12 across the transitory vessel 901.

Referring back to FIG. 9A, a stage S226 of flowchart 220 encompasses a determination if the virtual wall 15a was breached. If guidewire 11 had breached the virtual wall 15a, then flowchart 220 would terminate at stage S234.

Otherwise, if guidewire 11 contacted the physical wall of transitory vessel 902 as shown in FIG. 10A, then a stage S228 of flowchart 220 encompasses a retraction of guidewire 11 into guide catheter 12 and a stage S230 of flowchart 220 encompasses a determination if a linear direction of sample positions should be reversed. The linear direction is maintained for the first sample position as shown in FIG. 10A, and the flowchart 220 proceeds to loop through stages S222-S230 for the second through fifth sample positions as shown in respective FIGS. 10B-10E whereby a virtual wall boundary 15b is constructed.

Upon completion of the fifth sample position of FIG. 10E, stage S230 determines the linear direction of the sample positions should be reversed whereby guide catheter 12 is rotated within transitory vessel 901 to a sixth sample position as shown in FIG. 10F. Flowchart 200 to loop through stages S222-S230 for the sixth and eighth sample positions as shown in respective FIGS. 10F-10H whereby a virtual wall boundary 15a is breached by guidewire 11.

In practice, a detection of successful cannulation is assessed the previously described metric(s) being utilized. In case of the cannulation, the guidewire tends to follow the tubular constraints of the target vessel and is visually apparent. Consequently, computer vision methods can be used with standard Euclidean distance metrics for modeling the cannulation.

Alternatively in practice, an AI classifier may be used to determine the cannulation based on the shape of the guidewire and the behavior of the guide catheter. More particularly, an input into the deep learning classifier is the shape of the devices (e.g. as line segments or image) and the output is the likelihood that the guidewire entered the target vessel (based on the guidewire shape) vs contact with the wall preventing cannulation. A more robust version can use a sequence of shapes/images that encompass the cycle of the guidewire translating out of the guide catheter.

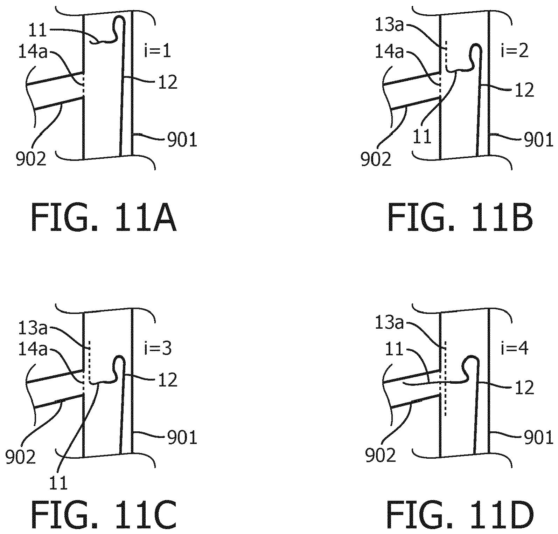

Referring back to FIG. 7B, FIGS. 11A-11D illustrate an example whereby flowchart 200 is modified to rotate guidewire 11 and guide catheter 12 upon an X-ray image showing guidewire 11 and guide catheter 12 are aligned with virtual entry way 14 as shown in FIGS. 11C and FIG. 11D. Guidewire 11 and guide catheter 12 will be incrementally rotated until guidewire 11 is detected entering target blood vessel 902 through the virtual entryway 14.

Referring back to FIG. 8, during the positional wall sampling, upon a detection by vessel cannulation module 83 of a traversal of guidewire 11 across the transitory blood vessel relative to catheter 12 into the target blood vessel through the virtual entryway of the virtual wall, positional wall sampling module 82 will command the interventional robot 110 to sequentially translate the guide 11 into the target blood vessel and translate catheter 12 into the target blood vessel. This traversal will occur at the sampling position corresponding to such detection.

Conversely, during the positional wall sampling, whenever vessel cannulation module 83 fails to detect a traversal of guidewire 11 across the transitory blood vessel relative to catheter 12 into the target blood vessel through the virtual entryway of the virtual wall, positional wall sampling module 82 will command the interventional robot 110 to rotate and/or translate guidewire 11 and catheter 12 within the transitory blood vessel.

Still referring to FIG. 8, during the positional wall sampling, whenever vessel cannulation module 83 fails to detect a traversal of guidewire 11 across the transitory blood vessel relative to catheter 12 into the target blood vessel through the virtual entryway of the virtual wall, positional wall sampling module 82 will command the interventional robot 110 to rotate guidewire 11 and catheter 12 to a predetermined orientation within the transitory blood vessel and then command X-ray imager 100 to generate an X-ray image of the transitory blood vessel in dependence upon the rotation of guidewire 11 and catheter 12 to the predetermined orientation within the transitory blood vessel. More particularly, X-ray imager 100 will set the necessary parameter(s), as known in the art of the present disclosure or hereinafter conceived, to facilitate an optical X-ray imaging of the blood vessels. For example, C-arm 130 may be moved to an orientation relative to the patient 100 that corresponds to the rotation of guidewire 11 and catheter 12 to a predetermined orientation within the transitory blood vessel.

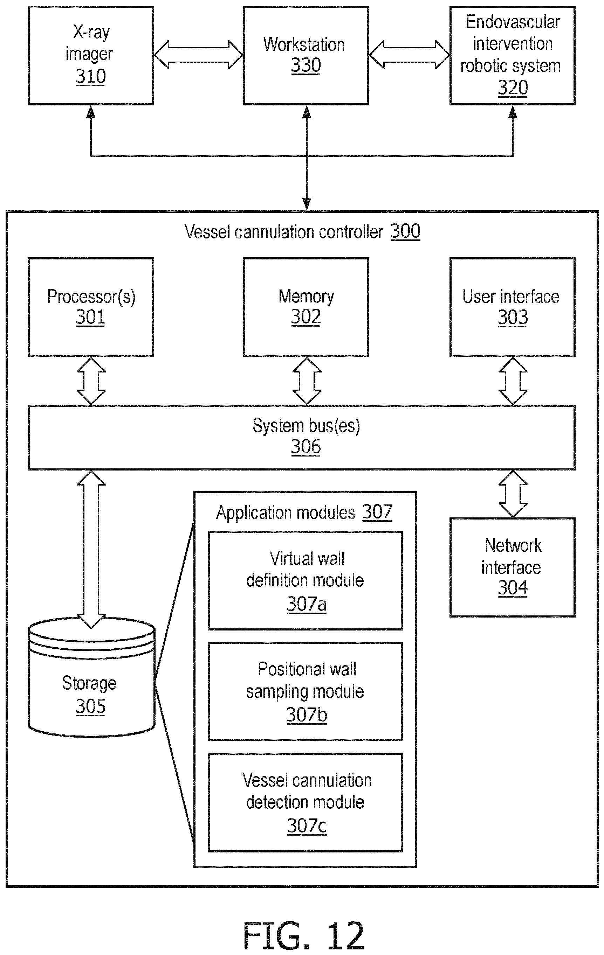

To facilitate a further understanding of the various inventions of the present disclosure, the following description of FIG. 12 teaches an exemplary embodiment of a vessel cannulation controller of the present disclosure. From this description, those having ordinary skill in the art will appreciate how to apply various aspects of the present disclosure for making and using additional embodiments of a vessel cannulation controller of the present disclosure.

Referring to FIG. 12, vessel cannulation controller 300 includes one or more processor(s) 301, memory 302, a user interface 303, a network interface 304, and a storage 305 interconnected via one or more system buses 306.

Each processor 301 may be any hardware device, as known in the art of the present disclosure or hereinafter conceived, capable of executing instructions stored in memory 302 or storage or otherwise processing data. In a non-limiting example, the processor(s) 301 may include a microprocessor, field programmable gate array (FPGA), application-specific integrated circuit (ASIC), or other similar devices.

The memory 302 may include various memories, as known in the art of the present disclosure or hereinafter conceived, including, but not limited to, L1, L2, or L3 cache or system memory. In a non-limiting example, the memory 302 may include static random access memory (SRAM), dynamic RAM (DRAM), flash memory, read only memory (ROM), or other similar memory devices.

The user interface 303 may include one or more devices, as known in the art of the present disclosure or hereinafter conceived, for enabling communication with a user such as an administrator. In a non-limiting example, the user interface may include a command line interface or graphical user interface that may be presented to a remote terminal via the network interface 304.

The network interface 304 may include one or more devices, as known in the art of the present disclosure or hereinafter conceived, for enabling communication with other hardware devices. In a non-limiting example, the network interface 304 may include a network interface card (NIC) configured to communicate according to the Ethernet protocol. Additionally, the network interface 304 may implement a TCP/IP stack for communication according to the TCP/IP protocols. Various alternative or additional hardware or configurations for the network interface 304 will be apparent.

The storage 305 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 305 may store instructions for execution by the processor(s) 301 or data upon with the processor(s) 301 may operate. For example, the storage 305 may store a base operating system for controlling various basic operations of the hardware.

The storage 305 also stores application modules 307 in the form of executable software/firmware for implementing the various functions of the controller 300 as previously described in the present disclosure including, but not limited to, a virtual wall definition module 307a, a positional wall sampling module 307b and a vessel cannulation detection module 307c.

In practice, vessel cannulation controller 300 may be (1) installed within an X-ray imager 310, (2) installed within an endovascular intervention robotic system 320 or (3) a stand-alone workstation 330 in communication with an X-ray imager 310 and endovascular intervention robotic system 320 (e.g., a client workstation or a mobile device like a tablet).

Alternatively, components of controller 300 may be distributed among X-ray imager 310, endovascular intervention robotic system 320 and/or stand-alone workstation 330.

Referring to FIGS. 1-12, those having ordinary skill in the art of the present disclosure will appreciate numerous benefits of the inventions of the present disclosure including, but not limited to, an improved endovascular intervention workflow by assisting in navigating an endovascular instrument from a transitory blood vessel into a target blood vessel branching from the transitory blood vessel by fluoroscopic imaging synchronized with servo control of the endovascular instrument.

Further, as one having ordinary skill in the art will appreciate in view of the teachings provided herein, structures, elements, components, etc. described in the present disclosure/specification and/or depicted in the Figures may be implemented in various combinations of hardware and software, and provide functions which may be combined in a single element or multiple elements. For example, the functions of the various structures, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the various and numerous inventions of the present disclosure (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device/system or such as may be used/implemented in/with a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A vessel cannulation system for a blood vessel cannulation of a target blood vessel by an endovascular instrument (10) navigational within a transitory blood vessel, the endovascular instrument (10) having a proximal section and a distal section, the vessel cannulation system comprising:
an interventional robot (110) connectable to the proximal section of the endovascular instrument (10),
wherein, when connected to the proximal section of the endovascular instrument (10), the interventional robot (110) being configured to navigate the distal section of the endovascular instrument (10) within the transitory blood vessel; and
a vessel cannulation controller (80) in control communication with the interventional robot (110),
wherein, within an image space encompassing the target blood vessel branching from the transitory blood vessel, the vessel cannulation controller (80) being configured to define a virtual wall of the transitory blood vessel having a virtual entryway into the target blood vessel;
wherein, when the interventional robot (110) is connected to the proximal section of the endovascular instrument (10), the vessel cannulation controller (80) is further configured to command the interventional robot (110) to execute a positional wall sampling of the transitory blood vessel by the distal section of the endovascular instrument (10); and
wherein the vessel cannulation controller (80) is further configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10).

2. The vessel cannulation system of claim 1,
wherein, within the image space, the vessel cannulation controller (80) being configured to define the virtual wall of the transitory blood vessel having the virtual entryway into the target blood vessel includes:
the vessel cannulation controller (80) configured to formulate at least one outlier of a set of wall sampling points as representing a location of the virtual entryway of the virtual wall within the image space;
wherein the vessel cannulation controller (80) being configured to command the interventional robot (110) to execute the positional wall sampling of the transitory blood vessel by the distal section of the endovascular instrument (10) includes:
the vessel cannulation controller (80) configured to derive the set of wall sampling points within the image space from a traversal of the distal section of the endovascular instrument (10) across the transitory vessel at various sampling positions with the transitory vessel; and
wherein the vessel cannulation controller (80) being configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes:
the vessel cannulation controller (80) configured to identify at least one sampling position corresponding to the at least one outlier based on a fitting of at least one line segment to the set of wall sampling points.

3. The vessel cannulation system of claim 1,
wherein, within the image space, the vessel cannulation controller (80) being configured to define the virtual wall of the transitory blood vessel having the virtual entryway into the target blood vessel includes:
the vessel cannulation controller (80) configured to formulate a force metric representing a location of the virtual entryway of the virtual wall within the image space;
wherein the vessel cannulation controller (80) being configured to command the interventional robot (110) to execute the positional wall sampling of the transitory blood vessel by the distal section of the endovascular instrument (10) includes:
the vessel cannulation controller (80) configured to derive a set of measured forces applied to the distal section of the endovascular instrument (10) within the image space from a traversal of the distal section of the endovascular instrument (10) across the transitory vessel at various sampling positions with the transitory vessel; and
wherein the vessel cannulation controller (80) being configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes:
the vessel cannulation controller (80) configured to identify at least one sampling position corresponding to the force metric based on the measured forces applied to the distal section of the endovascular instrument (10) during the positional wall sampling of the transitory blood vessel by the distal section of the endovascular instrument (10).

4. The vessel cannulation system of claim 1,
wherein, within the image space, the vessel cannulation controller (80) being configured to define the virtual wall of the transitory blood vessel having the virtual entryway into the target blood vessel includes:
the vessel cannulation controller (80) configured to formulate a distance metric representing a location of the virtual entryway of the virtual wall within the image space;
wherein the vessel cannulation controller (80) being configured to command the interventional robot (110) to execute the positional wall sampling of the transitory blood vessel by the distal section of the endovascular instrument (10) includes:
the vessel cannulation controller (80) configured to derive a set of measured navigated distances of the distal section of the endovascular instrument (10) within the image space from a traversal of the distal section of the endovascular instrument (10) across the transitory vessel at various sampling positions with the transitory vessel; and
wherein the vessel cannulation controller (80) being configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes:
the vessel cannulation controller (80) configured to identify at least one sampling position corresponding to the distance metric based on a derived set of navigated distances of the distal section of the endovascular instrument (10) during the positional wall sampling of the transitory blood vessel by the distal section of the endovascular instrument (10).

5. The vessel cannulation system of claim 1,
wherein, within the image space, the vessel cannulation controller (80) being configured to define the virtual wall of the transitory blood vessel having the virtual entryway into the target blood vessel includes:
the vessel cannulation controller (80) configured to formulate at least on shape profile of the endovascular instrument (10) representing a location of the virtual entryway of the virtual wall within the image space;
wherein the vessel cannulation controller (80) being configured to command the interventional robot (110) to execute the positional wall sampling of the transitory blood vessel by the distal section of the endovascular instrument (10) includes:
the vessel cannulation controller (80) configured to derive a set of measured shape profiles of the distal section of the endovascular instrument (10) within the image space from a traversal of the distal section of the endovascular instrument (10) across the transitory vessel at various sampling positions with the transitory vessel; and
wherein the vessel cannulation controller (80) being configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes:
the vessel cannulation controller (80) configured to identify at least one sampling position corresponding to the at least one shape profiles based on a derived set of shape profiles of the distal section of the endovascular instrument (10) during the positional wall sampling of the transitory blood vessel by the distal section of the endovascular instrument (10).

6. The vessel cannulation system of claim 1, wherein, within the image space, the vessel cannulation controller (80) being configured to define the virtual wall of the transitory blood vessel having the virtual entryway into the target blood vessel includes at least one of:
the vessel cannulation controller (80) configured to formulate at least one outlier of a set of wall sampling points as representing a location of the virtual entryway of the virtual wall within the image space;
the vessel cannulation controller (80) configured to formulate a force metric representing a location of the virtual entryway of the virtual wall within the image space;
the vessel cannulation controller (80) configured to formulate a distance metric representing a location of the virtual entryway of the virtual wall within the image space; and
the vessel cannulation controller (80) configured to formulate at least on shape profile of the endovascular instrument (10) representing a location of the virtual entryway of the virtual wall within the image space.

7. The vessel cannulation system of claim 1,
wherein the endovascular instrument (10) includes a co-axial alignment of an inner endovascular device (11) within an outer endovascular device (12);
wherein the vessel cannulation controller (80) being configured to command the interventional robot (110) to execute a positional wall sampling of the transitory blood vessel by the distal section of the endovascular instrument (10) includes:
the vessel cannulation controller (80) configured to command the interventional robot (110) to at least one of translate and rotate the inner endovascular device (11) and the outer endovascular device (12) to a wall sample position relative to the physical wall of the transitory blood vessel, and
at the wall sample position, the vessel cannulation controller (80) configured to command the interventional robot (110) to traverse the inner endovascular device (11) across the transitory blood vessel relative to the outer endovascular device (12); and
wherein the vessel cannulation controller (80) being configured to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes:
the vessel cannulation controller (80) configured to detect a traversal of the inner endovascular device (11) across the transitory blood vessel relative to the outer endovascular device (12) into the target blood vessel through the virtual entryway of the virtual wall.

8. The vessel cannulation system of claim 7, wherein, when the vessel cannulation controller (80) detects the traversal of the inner endovascular device (11) across the transitory blood vessel relative to the outer endovascular device (12) into the target blood vessel through the virtual entryway of the virtual wall, the vessel cannulation controller (80) is further configured to command the interventional robot (110) to sequentially translate the inner endovascular device (11) into the target blood vessel and translate the outer endovascular device into the target blood vessel.

9. The vessel cannulation system of claim 7, wherein, when the vessel cannulation controller (80) fails to detect a traversal of the inner endovascular device (11) across the transitory blood vessel relative to the outer endovascular device (12) into the target blood vessel through the virtual entryway of the virtual wall, the vessel cannulation controller (80) is further configured to command the interventional robot (110) to at least one of rotate and translate the inner endovascular device (11) and the outer endovascular device (12) within the transitory blood vessel.

10. The vessel cannulation system of claim 1, further comprising:
a medical imager (20) configured to generate at least one image of the transitory blood vessel and the target blood vessel; and
wherein, when the vessel cannulation controller (80) fails to detect the blood vessel cannulation of the target blood vessel by the distal section of the endovascular instrument (10) through the virtual entryway of the virtual wall during the positional wall sampling of the transitory blood vessel by the distal section of the endovascular instrument (10), the vessel cannulation controller (80) is configured to command the interventional robot (110) to rotate the endovascular instrument (10) to a predetermined orientation within the transitory blood vessel and to command the medical imager (20) to generate the image of the transitory blood vessel in dependence upon the rotation of the endovascular instrument (10) to the predetermined orientation within the transitory blood vessel.

11. A vessel cannulation controller (80), comprising:
at least one processor; and
a non-transitory machine-readable storage medium encoded with instructions for execution by the at least one processor of a blood vessel cannulation of a target blood vessel by an endovascular instrument (10) having a proximal section connected to an interventional robot (11) and a distal section navigational within a transitory blood vessel, wherein the non-transitory machine-readable storage medium includes instructions to:
define, within an image space encompassing the target blood vessel branching from the transitory blood vessel, a virtual wall of the transitory blood vessel having a virtual entryway into the target blood vessel;
command the interventional robot (110) to execute a positional wall sampling of the transitory blood vessel by the distal section of the endovascular instrument (10); and
detect the blood vessel cannulation of the target blood vessel by the distal section of the endovascular instrument (10) through the virtual entryway of the virtual wall during the positional wall sampling of the transitory blood vessel by the distal section of the endovascular instrument (10).

12. The vessel cannulation controller (80) of claim 11,
wherein instructions to define, within the image space, the virtual wall of the transitory blood vessel having the virtual entryway into the target blood vessel includes instructions to:
formulate at least one outlier of a set of wall sampling points as representing a location of the virtual entryway of the virtual wall within the image space;
wherein the instructions to command the interventional robot (110) to execute the positional wall sampling of the transitory blood vessel by the distal section of the endovascular instrument (10) includes instructions to:
derive the set of wall sampling points within the image space from a traversal of the distal section of the endovascular instrument (10) across the transitory vessel at various sampling positions with the transitory vessel; and
wherein instructions to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes instructions to:
identify at least one sampling position corresponding to the at least one outlier based on a fitting of at least one line segment to the set of wall sampling points.

13. The vessel cannulation controller (80) of claim 11,
wherein instructions to define, within the image space, the virtual wall of the transitory blood vessel having the virtual entryway into the target blood vessel includes instructions to:
formulate a force metric representing a location of the virtual entryway of the virtual wall within the image space;
wherein the instructions to command the interventional robot (110) to execute the positional wall sampling of the transitory blood vessel by the distal section of the endovascular instrument (10) includes instructions to:
derive a set of measured forces applied to the distal section of the endovascular instrument (10) within the image space from a traversal of the distal section of the endovascular instrument (10) across the transitory vessel at various sampling positions with the transitory vessel; and
wherein instructions to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes instructions to:
identify at least one sampling position corresponding to the force metric based on the measured forces applied to the distal section of the endovascular instrument (10) during the positional wall sampling of the transitory blood vessel by the distal section of the endovascular instrument (10).

14. The vessel cannulation controller (80) of claim 11,
wherein instructions to define, within the image space, the virtual wall of the transitory blood vessel having the virtual entryway into the target blood vessel includes instructions to:
formulate a distance metric representing a location of the virtual entryway of the virtual wall within the image space;
wherein the instructions to command the interventional robot (110) to execute the positional wall sampling of the transitory blood vessel by the distal section of the endovascular instrument (10) includes instructions to:
derive a set of measured navigated distances of the distal section of the endovascular instrument (10) within the image space from a traversal of the distal section of the endovascular instrument (10) across the transitory vessel at various sampling positions with the transitory vessel; and
wherein instructions to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes instructions to:
identify at least one sampling position corresponding to the distance metric based on a derived set of navigated distances of the distal section of the endovascular instrument (10) during the positional wall sampling of the transitory blood vessel by the distal section of the endovascular instrument (10).

15. The vessel cannulation controller (80) of claim 11,
wherein instructions to define, within the image space, the virtual wall of the transitory blood vessel having the virtual entryway into the target blood vessel includes instructions to:
formulate at least on shape profile of the endovascular instrument (10) representing a location of the virtual entryway of the virtual wall within the image space;
wherein the instructions to command the interventional robot (110) to execute the positional wall sampling of the transitory blood vessel by the distal section of the endovascular instrument (10) includes instructions to:
derive a set of measured shape profiles of the distal section of the endovascular instrument (10) within the image space from a traversal of the distal section of the endovascular instrument (10) across the transitory vessel at various sampling positions with the transitory vessel; and
wherein instructions to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes instructions to:
identify at least one sampling position corresponding to the at least one shape profiles based on a derived set of shape profiles of the distal section of the endovascular instrument (10) during the positional wall sampling of the transitory blood vessel by the distal section of the endovascular instrument (10).

16. The vessel cannulation controller (80) of claim 11, wherein the instructions to define, within the image space, the virtual wall of the transitory blood vessel having the virtual entryway into the target blood vessel includes instructions to at least one of:
formulate at least one outlier of a set of wall sampling points as representing a location of the virtual entryway of the virtual wall within the image space;
formulate a force metric representing a location of the virtual entryway of the virtual wall within the image space;
formulate a distance metric representing a location of the virtual entryway of the virtual wall within the image space; and
formulate at least on shape profile of the endovascular instrument (10) representing a location of the virtual entryway of the virtual wall within the image space.

17. The vessel cannulation controller (80) of claim 11,
wherein the endovascular instrument (10) includes a co-axial alignment of an inner endovascular device (11) within an outer endovascular device (12);
wherein the instructions to command the interventional robot (110) to execute a positional wall sampling of the transitory blood vessel by the distal section of the endovascular instrument (10) includes instructions to:
command the interventional robot (110) to at least one of translate and rotate the inner endovascular device (11) and the outer endovascular device (12) to a wall sample position relative to the physical wall of the transitory blood vessel, and
at the wall sample position, command the interventional robot (110) to traverse the inner endovascular device (11) across the transitory blood vessel relative to the outer endovascular device (12); and
wherein the instructions to detect the blood vessel cannulation of the target blood vessel by the distal section of the endovascular instrument (10) through the virtual entryway of the virtual wall during the positional wall sampling of the transitory blood vessel by the distal section of the endovascular instrument (10) includes instructions to:
detect a traversal of the inner endovascular device (11) across the transitory blood vessel relative to the outer endovascular device (12) into the target blood vessel through the virtual entryway of the virtual wall.

18. The vessel cannulation controller (80) of claim 17, wherein, when the vessel cannulation controller (80) detects the traversal of the inner endovascular device (11) across the transitory blood vessel relative to the outer endovascular device (12) into the target blood vessel through the virtual entryway of the virtual wall, the non-transitory machine-readable storage medium further includes instructions to command the interventional robot (110) to sequentially translate the inner endovascular device (11) into the target blood vessel and translate the outer endovascular device into the target blood vessel.

19. The vessel cannulation controller (80) of claim 17, wherein, when the vessel cannulation controller (80) fails to detect a traversal of the inner endovascular device (11) across the transitory blood vessel relative to the outer endovascular device (12) into the target blood vessel through the virtual entryway of the virtual wall, the non-transitory machine-readable storage medium further includes instructions to command the interventional robot (110) to at least one of rotate and translate the inner endovascular device (11) and the outer endovascular device (12) within the transitory blood vessel.

20. The vessel cannulation controller (80) of claim 11, wherein, when the vessel cannulation controller (80) fails to detect the blood vessel cannulation of the target blood vessel by the distal section of the endovascular instrument (10) through the virtual entryway of the virtual wall during the positional wall sampling of the transitory blood vessel by the distal section of the endovascular instrument (10), the non-transitory machine-readable storage medium further includes instructions to command the interventional robot (110) to rotate the endovascular instrument (10) to a predetermined orientation within the transitory blood vessel and to command a medical imager (20) to generate an image of the transitory blood vessel in dependence upon the rotation of the endovascular instrument (10) to the predetermined orientation within the transitory blood vessel.

21. A vessel cannulation method executable by a vessel cannulation controller (80) for a blood vessel cannulation of a target blood vessel by an endovascular instrument (10) having a proximal section connected to an interventional robot (11) and a distal section navigational within a transitory blood vessel, wherein the vessel cannulation method comprises the vessel cannulation controller (80):
defining, within an image space encompassing the target blood vessel branching from the transitory blood vessel, a virtual wall of the transitory blood vessel having a virtual entryway into the target blood vessel;
commanding the interventional robot (110) to execute a positional wall sampling of the transitory blood vessel by the distal section of the endovascular instrument (10); and
detecting the blood vessel cannulation of the target blood vessel by the distal section of the endovascular instrument (10) through the virtual entryway of the virtual wall during the positional wall sampling of the transitory blood vessel by the distal section of the endovascular instrument (10).

22. The vessel cannulation method of claim 21,
wherein the defining within the image space, by the vessel cannulation controller (80), of the virtual wall of the transitory blood vessel having the virtual entryway into the target blood vessel includes the vessel cannulation controller (80):
formulating at least one outlier of a set of wall sampling points as representing a location of the virtual entryway of the virtual wall within the image space;
wherein the commanding, by the vessel cannulation controller (80), the interventional robot (110) to execute the positional wall sampling of the transitory blood vessel by the distal section of the endovascular instrument (10) includes the vessel cannulation controller (80):
deriving the set of wall sampling points within the image space from a traversal of the distal section of the endovascular instrument (10) across the transitory vessel at various sampling positions with the transitory vessel; and
wherein the detecting, by the vessel cannulation controller (80), the blood vessel cannulation of the target blood vessel by the distal section of the endovascular instrument (10) through the virtual entryway of the virtual wall during the positional wall sampling of the transitory blood vessel by the distal section of the endovascular instrument (10) includes the vessel cannulation controller (80):
identify at least one sampling position corresponding to the force metric based on a derived set of measured forces applied to the distal section of the endovascular instrument (10) during the positional wall sampling of the transitory blood vessel by the distal section of the endovascular instrument (10).

23. The vessel cannulation method of claim 21,
wherein the defining within the image space, by the vessel cannulation controller (80), of the virtual wall of the transitory blood vessel having the virtual entryway into the target blood vessel includes the vessel cannulation controller (80):
formulating a force metric representing a location of the virtual entryway of the virtual wall within the image space;
wherein the commanding, by the vessel cannulation controller (80), the interventional robot (110) to execute the positional wall sampling of the transitory blood vessel by the distal section of the endovascular instrument (10) includes the vessel cannulation controller (80):
derive a set of measured forces applied to the distal section of the endovascular instrument (10) within the image space from a traversal of the distal section of the endovascular instrument (10) across the transitory vessel at various sampling positions with the transitory vessel; and
wherein the detecting, by the vessel cannulation controller (80), the blood vessel cannulation of the target blood vessel by the distal section of the endovascular instrument (10) through the virtual entryway of the virtual wall during the positional wall sampling of the transitory blood vessel by the distal section of the endovascular instrument (10) includes the vessel cannulation controller (80):
identifying at least one sampling position corresponding to the force metric based on a derived set of measured forces applied to the distal section of the endovascular instrument (10) during the positional wall sampling of the transitory blood vessel by the distal section of the endovascular instrument (10).

24. The vessel cannulation method of claim 21,
wherein the defining within the image space, by the vessel cannulation controller (80), of the virtual wall of the transitory blood vessel having the virtual entryway into the target blood vessel includes the vessel cannulation controller (80):
formulating a distance metric representing a location of the virtual entryway of the virtual wall within the image space;
wherein the commanding, by the vessel cannulation controller (80), the interventional robot (110) to execute the positional wall sampling of the transitory blood vessel by the distal section of the endovascular instrument (10) includes the vessel cannulation controller (80):
derive a set of navigated distances of the distal section of the endovascular instrument (10) within the image space from a traversal of the distal section of the endovascular instrument (10) across the transitory vessel at various sampling positions with the transitory vessel; and
wherein the detecting, by the vessel cannulation controller (80), the blood vessel cannulation of the target blood vessel by the distal section of the endovascular instrument (10) through the virtual entryway of the virtual wall during the positional wall sampling of the transitory blood vessel by the distal section of the endovascular instrument (10) includes the vessel cannulation controller (80):
identifying at least one sampling position corresponding to the distance metric based on a derived set of navigated distances of the distal section of the endovascular instrument (10) during the positional wall sampling of the transitory blood vessel by the distal section of the endovascular instrument (10).

25. The vessel cannulation method of claim 21,
wherein the defining within the image space, by the vessel cannulation controller (80), of the virtual wall of the transitory blood vessel having the virtual entryway into the target blood vessel includes the vessel cannulation controller (80):
formulating at least on shape profile of the endovascular instrument (10) representing a location of the virtual entryway of the virtual wall within the image space;
wherein the commanding, by the vessel cannulation controller (80), the interventional robot (110) to execute the positional wall sampling of the transitory blood vessel by the distal section of the endovascular instrument (10) includes the vessel cannulation controller (80):
deriving a set of measured shape profiles of the distal section of the endovascular instrument (10) within the image space from a traversal of the distal section of the endovascular instrument (10) across the transitory vessel at various sampling positions with the transitory vessel; and
wherein the, by the vessel cannulation controller (80), the blood vessel cannulation of the target blood vessel by the distal section of the endovascular instrument (10) through the virtual entryway of the virtual wall during the positional wall sampling of the transitory blood vessel by the distal section of the endovascular instrument (10) includes the vessel cannulation controller (80):
identifying at least one sampling position corresponding to the at least one shape profiles based on a derived set of shape profiles of the distal section of the endovascular instrument (10) during the positional wall sampling of the transitory blood vessel by the distal section of the endovascular instrument (10).

26. The vessel cannulation method of claim 21, wherein the defining within the image space, by the vessel cannulation controller (80), of the virtual wall of the transitory blood vessel having the virtual entryway into the target blood vessel includes the vessel cannulation controller (80):
at least one of:
formulating at least one outlier of a set of wall sampling points as representing a location of the virtual entryway of the virtual wall within the image space;
formulating a force metric representing a location of the virtual entryway of the virtual wall within the image space;
formulating a distance metric representing a location of the virtual entryway of the virtual wall within the image space; and
formulating at least on shape profile of the endovascular instrument (10) representing a location of the virtual entryway of the virtual wall within the image space.

27. The vessel cannulation method of claim 21,
wherein the endovascular instrument (10) includes a co-axial alignment of an inner endovascular device (11) within an outer endovascular device (12);
wherein the commanding, by the vessel cannulation controller (80), the interventional robot (110) to execute the positional wall sampling of the transitory blood vessel by the distal section of the endovascular instrument (10) includes the vessel cannulation controller (80):
commanding the interventional robot (110) to at least one of translate and rotate the inner endovascular device (11) and the outer endovascular device (12) to a wall sample position relative to the physical wall of the transitory blood vessel, and
at the wall sample position, commanding the interventional robot (110) to traverse the inner endovascular device (11) across the transitory blood vessel relative to the outer endovascular device (12); and
wherein the detecting, by the vessel cannulation controller (80), the blood vessel cannulation of the target blood vessel by the distal section of the endovascular instrument (10) through the virtual entryway of the virtual wall during the positional wall sampling of the transitory blood vessel by the distal section of the endovascular instrument (10) includes the vessel cannulation controller (80):
detecting a traversal of the inner endovascular device (11) across the transitory blood vessel relative to the outer endovascular device (12) into the target blood vessel through the virtual entryway of the virtual wall.

28. The vessel cannulation method of claim 27, further comprising, when the vessel cannulation controller (80) detects the traversal of the inner endovascular device (11) across the transitory blood vessel relative to the outer endovascular device (12) into the target blood vessel through the virtual entryway of the virtual wall, the vessel cannulation controller (80):
commanding the interventional robot (110) to sequentially translate the inner endovascular device (11) into the target blood vessel and translate the outer endovascular device into the target blood vessel.

29. The vessel cannulation method of claim 27, further comprising, when the vessel cannulation controller (80) fails to detect the traversal of the inner endovascular device (11) across the transitory blood vessel relative to the outer endovascular device (12) into the target blood vessel through the virtual entryway of the virtual wall, the vessel cannulation controller (80):
commanding the interventional robot (110) to at least one of rotate and translate the inner endovascular device (11) and the outer endovascular device (12) within the transitory blood vessel.

30. The vessel cannulation method of claim 21, further comprising, when the vessel cannulation controller (80) fails to detect the blood vessel cannulation of the target blood vessel by the distal section of the endovascular instrument (10) through the virtual entryway of the virtual wall during the positional wall sampling of the transitory blood vessel by the distal section of the endovascular instrument (10), the vessel cannulation controller (80):
commanding the interventional robot (110) to rotate the endovascular instrument (10) to a predetermined orientation within the transitory blood vessel; and
commanding a medical imager (20) to generate an image of the transitory blood vessel in dependence upon the rotation of the endovascular instrument (10) to the predetermined orientation within the transitory blood vessel.
